Europäisches Patentamt

European Patent Office

Office européen des brevets

(19)

(11) Publication number: **0 106 536**
**A2**

(12) # EUROPEAN PATENT APPLICATION

(21) Application number: 83305375.4

(22) Date of filing: 14.09.83

(51) Int. Cl.³: **G 01 N 33/54**
**G 01 N 21/07, B 04 B 5/04**

(30) Priority: 15.09.82 US 418493

(43) Date of publication of application:
25.04.84 Bulletin 84/17

(84) Designated Contracting States:
AT BE CH DE FR GB IT LI LU NL SE

(71) Applicant: ORTHO DIAGNOSTIC SYSTEMS INC.
One Johnson & Johnson Plaza
New Brunswick New Jersey 08933(US)

(72) Inventor: Teipel, John William
10 Meadow Run Road
Skillman New Jersey 08558(US)

(74) Representative: Jones, Alan John et al,
CARPMAELS & RANSFORD 43 Bloomsbury Square
London, WC1A 2RA(GB)

(54) Carousel microparticle separating, washing and reading device and method of use.

(57) A carousel microparticle separating, washing and reading device for application in solid-phase heterogeneous immunoassay systems and methods of use. After suitable immunological reaction in the microparticle containing sample-immunoassay mixture, the application of centrifugal force causes the particles to move through a washing solution and into a detection area where labelled microparticles may be read directly or, following enzymatic reaction of the microparticles with a substrate solution and the consequent formation of product, the product may be detected.

FIG-5a

FIG-5b SUBSTRATE SOLUTION ADDITION

FIG-5c WASH SOLUTION ADDITION

FIG-5d SAMPLE MIXTURE ADDITION

FIG-5e SEPARATING, WASHING AND READING

EP 0 106 536 A2

## CAROUSEL MICROPARTICLE SEPARATING, WASHING AND READING DEVICE AND METHOD OF USE

### Field of the Invention

This invention relates to apparatus and methods for the separation, washing and detection of microsphere type particles employed in immunoassay techniques.

### Background of the Invention

As is well known in the art, many diagnostically useful reagents can be made utilizing biologically active molecules.  The specificity of the reaction between immunoglobulins, antibodies, and the antigens stimulating their formation enhances the usefulness of such reagents. For example, immunoglobulins may be used to identify the presence of antigens in aqueous samples as well as their presence on the surfaces of substrates and tissue cells. As is readily know with such reactions, it is often necessary to enhance the reaction in order to afford greater sensitivity in its detection.

Numerous enhancement techniques have been utilized and generally are directed towards the attachment of a more easily detected marker substance to the biologically active material.  Thus, natural and synthetic microsphere forming polymers (also microparticles hereafter), microparticles, red blood cells, enzymes, iodophores and fluorochromes have been directly attached to immunoglobulins or their counterpart antigens. Alternately, immunoglobulins have been attached to natural and synthetic microsphere forming polymers in the so-called heterogeneous, non-isotopically labeled immunoassays as such techniques are commonly understood in the field of immunoassays generally.

Since the attachment of immunoglobulins to microparticles characteristically permits easier detection than detection of the immunoglobulin or its immunologically reactive antigen without such a microparticle, their employment provides generally increased sensitivity and/or more easily performed methods of detection.

Detection of an antigen may thus be accomplished by any one of several conventional immunoassay techniques using an immunologically reactive, i.e., complimentary, immunoglobulin coated microparticle. Conversely, if the presence of immunologically reactive antisera is to be detected in the sample, then antigen coated microparticles are utilized. Alternate indirect immunoassay methods may employ any number of intermediate, reactive immunoglobulins between the substance to be detected and the microparticle. The microparticles shall herein be alternatively termed the solid phase component. This invention is directed specifically to those immunoassays utilizing microparticles including the so-called microspheres, red blood cells and the like, as a necessary component of the assay.

Following the immunological reaction in so-called heterogeneous type assays, it is necessary to remove the solid phase and the attached immunological complex of antigen and antibody. As has been described in European Patent Publication No. 72,201 entitled Particle Washing System And Method Of Use and incorporated herein by reference, several washing procedures are typically required in order to collect and wash the particles (i.e. the solid phase component) and remove contaminating agents originating from the sample suspending solution. Since one of the major considerations in selecting an immunoassay technique for routine use is the number of required manipulations and related economic considerations, the reduction of mechanical manipulations is highly desirable. This becomes especially important when throughput considerations are paramount.

It is therefore an object of the present invention to provide apparatus and methods useful for multiple, simultaneous immunoassay procedures whereby the solid phase components, i.e., the microparticles having attached thereto the serologically reacted determinants, are separated from the original reaction mixture, are washed during their separation and, are reacted with a subsequent substrate as necessary (i.e., not necessary if a fluorescent or other such label is attached to one of the serologically reacted determinants in turn attached to the microparticles) to provide a reaction product capable of facile detection; all of the above procedures to take place in a "one step" washing, separating, and reacting procedure.

Although there are many types of conventional separation devices available, typically, they are intended to facilitate recovery of the mother solution portion of the suspension, for example, the recovery of blood plasma from a whole blood sample, and none is intended to solve the problems associated with providing a 'one-step' washing, separating and reacting procedure useful in immunoassays.

For example, U.S. Patent No. 4,244,694 to Farina et al. describes the use of a reactor/separator device for use in automated solid phase immunoassays. The described device employs a water impermeable disc capable of supporting immunoabsorbents, immobilized antisera, ion exchange resins and other similar materials for reaction with reagents added to the inner tube upon centrifugation. Following the desired reaction, additional centrifugal forces are applied in order to force the aqueous phase through the filter making it water permeable thus permitting separation of desired components. Farina's invention provides a device wherein centrifugal force is employed for the mixing, transferance and separation of soluble reactants in a reactor cavity separated from the collection chamber by a water impermeable disc. Such a device fails to solve the problems enumerated above, specifically those related to the collection, washing and subsequent reaction with substrate of microparticles suspended in a solution where a minimum of steps and a maximization of economy is desired.

U.S. Patent No. 3,953,172 to Shapiro et al. teaches methods and apparatus for assaying liquid materials. The apparatus described is designed to handle a plurality of samples which are subsequently separated by centrifugation through a chromatograph gel. Consequently, applications will be limited to labels such as iodophors, dyes including fluorochromes and enzyme labels but will not be effective when microparticles are being employed. Consequently, Shapiro et al.'s teachings will not be effective in overcoming the immunoassay problems associated with solid phase heterogeneous techniques.

It is an object of the present invention to provide methods for the separation, washing and subsequent reaction of microparticles in a solid phase heterogeneous immunoassay technique whereby multiple samples may be handled simultaneously in a "one step" mechanical operation.

Conventional types of apparatus for handling multiple samples include multiple cuvette rotors such as that described by Tiffany et al. in U.S. Patent No. 4,226,531. Described therein is a rotor having multiple cuvettes radially arranged about a center, each cuvette having adjoining sample and reagent/measuring chambers separated by a wedge shaped dam. The sample and reagent are mixed under increased gravitational force and light scattering or fluorescence is measured through the optical window at the outer wall of each cuvette. The cuvette designed is appropriate for homogeneous immunoassay techniques but fails to teach use of a multiple cuvette rotor for heterogeneous immunoassay methods which require the separation, washing and subsequent reaction of microparticles from a reaction sample. It is an object of the present invention to provide such a method whereby a multiple cuvette rotor of appropriate design may be used to rapidly and efficiently effect the separation and washing of microparticles from the reaction sample and their subsequent reaction with substrate permitting optical detection and measurement of reaction.

It is an object of the present invention to permit the rapid separation, washing, and detection by subsequent reaction with a substrate as necessary (i.e., not necessary if a labeled component is employed in the immunoassay), of microparticles from an immunoassay reaction sample solution in a "one step" mechanical operation. It is yet another objective that the original sample solution be separately maintained from the resulting microparticle substrate mixture to permit detection of the microparticle or the measurement of product formed in the substrate mixture without contaminating components of the original sample. It is still another objective of the present invention that these objectives be accomplished in a simple system capable of economical production and employable within inexpensive centrifuges commonly available. It is a further objective of the present invention to not only provide devices whereby the objectives may be accomplished but also methodology capable of meeting the desired objectives. These and other objectives will readily become apparent to those skilled in the art in the light of the teachings set forth herein.

## Summary of the Invention

In accordance with the principles and objectives of the present invention, apparatus is provided comprising a substantially circular carousel having a plurality of radially dispersed chambers about its center, said chambers having a closed end distal to the center and an open end proximal to the center. The open end communicates with a feed area which is disposed substantially at the center of the carousel. The entire apparatus is adapted for rotation about the center by a centrifuge. Typically, the adjacent chambers may share a common wall and in a preferred embodiment, the device further comprises barrier means which are disposed within the chamber substantially perpendicular to the radius of the carousel and communicating with the walls of the chamber. These barrier means are placed and dimensioned appropriately for stabilizing the washing and substrate solutions and for permitting passage of the microparticles.

Additionally, a sample holder means is provided for the separate mixing and incubation of the sample and immunoassay reagents under appropriate conditions and subsequent placement at substantially the center of the carousel device so that upon the application of sufficient centrifugal force, the microparticles from the immunoassay sample are applied to the wash and/or substrate solutions. The previously added wash and substrate solutions (these may be one and the same) are substantially maintained in place by the continuous application of centrifugal force which may, in one embodiment be assisted by a barrier. The centrifugal force will also act upon the microparticles contained within the immunoassay sample solution and force them to move through the wash solution and into the distal end of the chamber where the microparticles may be read directly, i.e. by sensing the amount of fluorescence or other label attached thereto, its light scattering effects or by measuring the production of a product from the substrate solution.

The wash solution is advantageously chosen to have a density less than that of the microparticles to be separated but more than that of the sample solution containing the microparticles so as to minimize physical mixing between the two solutions. Similar density limitations apply to the substrate solution, specifically, the substrate solution may have a density approaching that of the microparticles but no less than that of the wash solution. For economic reasons, it may be preferable to have just one solution of larger volume to serve both as a wash solution and as a substrate solution if required. This would reduce the number of different solutions required as well as simply the design of the carousel and the carousel centrifuge/pipetting machine.

For the methods and systems provided for separating, washing and detecting microparticles in an immunoassay system employing circular carousels, the preferred wash solution has at least one component for adjusting the density selected from the group consisting of sodium chloride, glycine, sugars, serum albumin, natural polymers, natural copolymers, synthetic polymers, synthetic copolymers, dextran and Ficoll". The serum albumin may be selected from the group consisting of animal serum albumin and human serum albumin. As is well-known, serum albumin, to be compatible with most immunoassays, cannot have gamma globulin or complement. If red blood cells are employed as the microparticles, then the wash solution must be selected so that it does not lyse the red blood cells.

0106536

## Brief Description of the Drawings

The objectives and principles of the invention and the preferred embodiments thereof will best be understood by reference to the accompanying drawings wherein:

Fig. 1 is a top view of the preferred embodiment of the microparticle, separating, washing, reacting and reading device.

Fig. 2 is a side view of the device shown in Fig. 1.

Fig. 3 is a top view of an embodiment of a sample holder for the device of Fig. 1.

Fig. 4 is a side view of the sample holder of Fig. 3.

Figs. 5 a-e show a side view of the preferred embodiment in operation.

Fig. 6 is a top view of the preferred embodiment of the stabilizing barrier.

Fig. 7 is a side view of the barrier of Fig. 6.

Figs. 8 a-c illustrate the principles of the present invention.

Fig. 9 is a side view of the most preferred embodiment of the rotor showing various chambers and barriers.

Fig. 10 is another side view of the most preferred embodiment shown during operation.

ORD-39

## Best Mode for Carrying Out the Invention

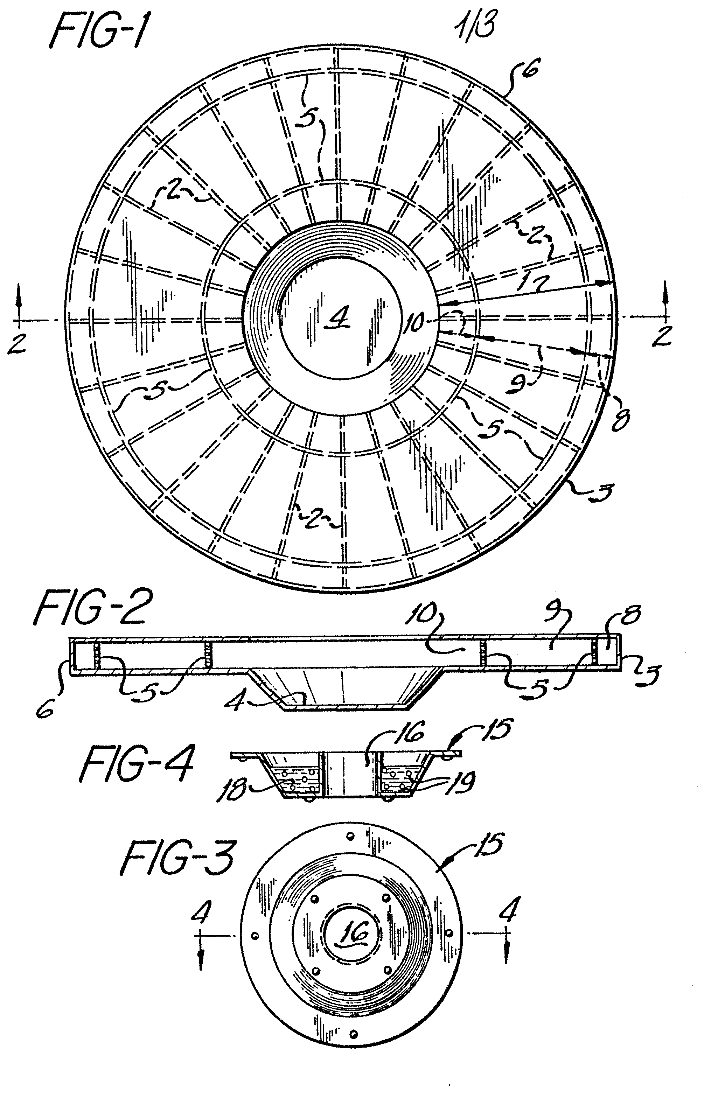

Illustrated in Figure 1 is an embodiment of the invention showing a plurality of radially dispersed chambers 1 forming an essentially circular carousel 3 having a center 25. The chambers have a distal closed end 6 and a proximal open end 7 which communicates with feed area 4. The preferred embodiment will have means, such as at least one barrier 5, for stabilizing the solutions and minimizing mechanical mixing therebetween.

The chamber may be described as having three areas within. The area most distal to the center, reaction area 8, contains the substrate solution. The microparticles will be read in this area as shown in Figure 2 and the substrate solution shall be selected in accordance with the method of reading employed. For example, the microparticles may be read directly, i.e. by measuring their fluorescence or effect on light scattering or other readily detectable label. Generally, such immunoassay techniques rely on multiple antibodies of differing specificities, some of which may be labeled with a market substance. These techniques are well known and those skilled in the art will readily understand that for the present invention to be useful in detecting the presence or absence of a substance, for example an antigen or antibody, in a sample, a relationship must be established between the level of labeled and unlabeled microparticles at the carousel reading cite, and the level of substance in the sample. The selection of antibodies or antigens and labels will thus be in accordance with the above and pursuant to the type of immunoassay technique employed as will be apparent to one skilled in the art.

Alternately to using a label, one may instead measure a product produced by the reactions of the microparticles on a substrate present in the substrate solution. Typically, the product to be read or the label to be detected will depend upon the type of immunoassay being performed and the level of sensitivity desired.

With further reference to Fig. 1, before the microparticles can be read in area 8, they must be washed of contaminents, such as unreacted-free label and the like from the sample. This is accomplished by passage of the microparticles through a wash solution. Intermediate area 9 of chamber 1 is reserved for the washing process. The wash solution shall typically be chosen to have a density greater than = equal to the density of the sample solution but less than that of the microparticles. Passage of the microparticles through the wash solution from sample area 10, centrally located on the carousel, in response to centrifugal force, results in the removal and dilution of contaminating components from the sample-immunoassay mixture solution.

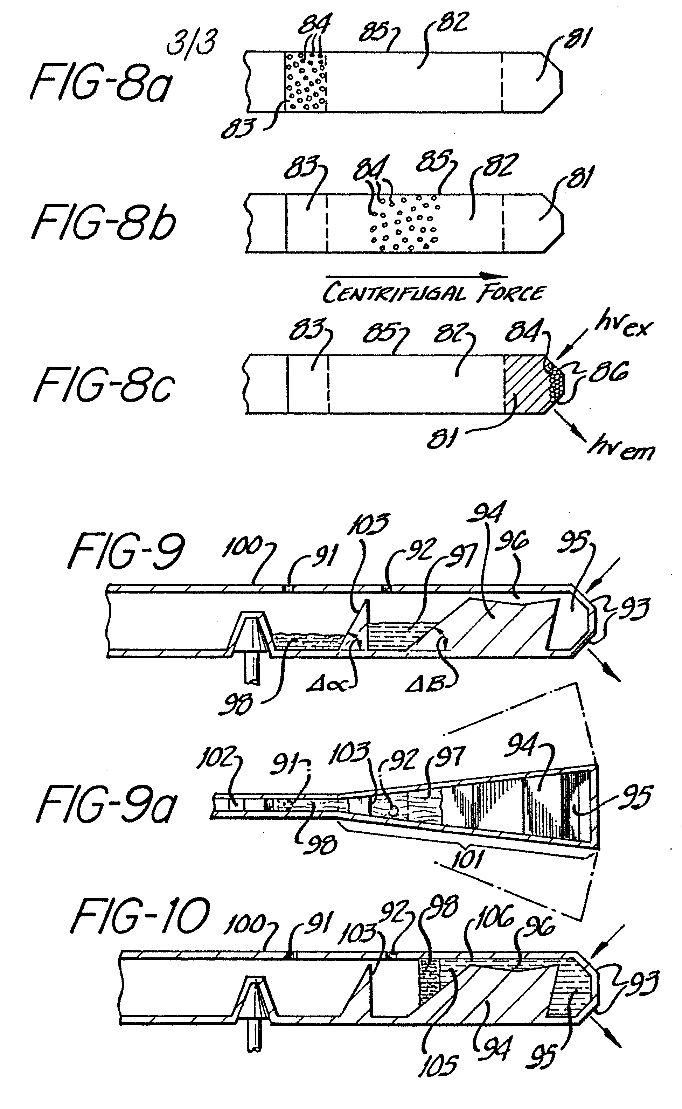

Figure 8 illustrates the principles involved particularly with regard to the effects of the washing solution. The rotors are illustrated in gross simplification without any types of barriers or other stabilizing means and it is assumed that with such an embodiment, layering of the different solutions is accomplished while the rotor 85 is spinning thereby forcing by centrifugal force the outward positioning of each layer of solution. Figure 8a shows a starting position after the substrate solution 81, wash solution 82, and reaction or sample mixture 83 has been applied. Under continued centrifugal force, the microparticles 84 contained within the sample mixture 83 travel through wash solution 82 which effectively dilutes any solute drag or other contaminants from the reaction mixture 83. The wash solution 82 is chosen to have a density no more than that of the microparticles and preferably greater than that of the reaction mixture 83 in order to limit physical admixing. Eventually, the microparticles will sediment after passage through substrate solution 81, shown is Figure 8c. The cross-hatching illustrates the enzymatic conversion of substrate in substrate solution 81 to a fluorescent product. The fluorescent product may be measured or detected through optical windows 86 by irradiating the substrate solution and measuring the effect of such irradiation.

With reference again to Figure 3, shown is the top view of a preferred embodiment of the sample holder 15 which may be placed in the feed area 4 of the carousel 3. The sample holder 15 conveniently contains the sample and immunoassay components for placement in the appropriate environment for stimulating the immunological reactions desired. The holder 15 is preferably constructed to have a central area 16 through which the substrate solution and wash solution may be added once the holder has been placed on the carousel and while the carousel is being rotated by a centrifuge. The carousel and holder are constructed so that solutions added to the feed area do not come into contact with the sample and flow into the chambers. Walls of holder 15 are designed appropriately so that under a diminished rotational speed, the substrate and washing solutions may be layered into the chamber and maintained there by the centrifugal force generated at the diminished speed and then, upon increasing the rotational speed and therefore the centrifugal force, sample 18 will be caused to overflow the holder walls and pass into sample area 10 in chamber 1. Thereafter, centrifugal force is appropriately selected in order to adjust the speed of the passage of the microparticles through the sample solution, the wash solution, and into the substrate solution as desired.

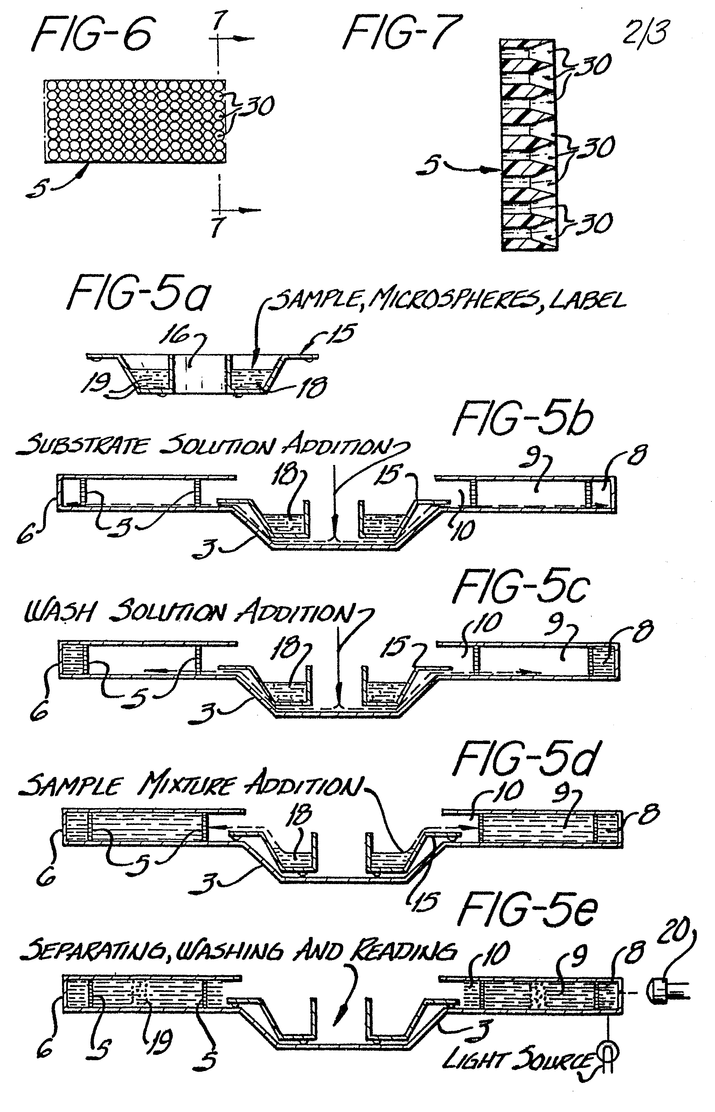

Figures 5a through 5e demonstrate the principles and application of the invention. Initially, the determinant containing sample, microparticles and label are added to sample holder 15 and the resulting sample-immunoassay mixture 18 is incubated or treated as required. The sample holder 15 with sample-immunoassay mixture 18 is then deposited in the feed area of carousel 3 and the carousel is rotated at a speed insufficient to cause the mixture to overflow the holder walls.

While the rotational speed is still limited, the substrate solution is added through central portion 16. The centrifugal force generated by rotation is directed radially outwards towards substrate area 8 and in response thereto, the substrate solution will flow to the distal end of the chamber, reaction area 8. Thereafter, the wash solution is added and similarly, it flows into chamber area 9 as shown in Figure 5c. Increasing the rotational speed results in application of greater centrifugal force urging the sample-immunoassay mixture 18 to leave sample holder 15 and layer itself adjacent to the wash solution as shown in Figure 5d.

Continuous centrifugal force causes the microparticles 19 to leave the sample mixture, pass through wash solution in area 9 and eventually into the substrate solution in area 8 as shown in Figure 5e. Concurrently, in one example of application, substrate area 8 may be excited by a light excitation source and resulting fluorescent radiation sensed by detector 20 as shown in Figure 5e. Entrance of the microparticles 19 into the substrate area 8 will advantageously result in variation of the resulting radiation thereby changing the detector signal which may be directly correlated to the presence of the immunological determinant in the sample. It is to be understood that the type and location of the radiation source and detection are not limited to the physical arrangement shown.

Figure 6 shows a top view of a preferred embodiment of the stabilizing barrier 5 having pores 30. The side view, shown in Figure 7, illustrates the construction of the barrier in order to promote stabilization of the solutions within the chambers of the carousel 3. As illustrated, it is preferred that the barrier have channels in the neighborhood of 1-5 millimeters and a pore diameter of approximately 20-100 microns. It is readily apparent that, to be operative, the pore diameter can not be substantially smaller than the diameter of the microparticles.

Figures 9 and 9a illustrate the side and top view respectively of the most preferred embodiment of a carousel rotor device. It is believed that the embodiment of these figures and Figure 10 is more readily adaptable to continuous operation in a centrifuge/pipetting machine. Although Figure 9a illustrates the chamber 101 of rotor 100 to have an increasing width as the distance from center 102 increases, it will be readily understood that alternatives such as constant width may be readily substituted. Figure 9 illustrates a side view of rotor 100 having read chamber 95, washing chamber 96, wash fluid reservoir 97, and sample fluid reservoir 98. Between sample reservoir 98 and wash fluid reservoir 97 is barrier 103 having a slope contiguous to the sample reservoir with an angle alpha. The barrier 103 maintains the sample and wash fluids separate but is designed to permit, under great centrifugal force, the escape of sample fluid over the top thereof. Similarly, wash fluid in chamber 97 is held in place by barrier 94 also having a slope but at an angle beta less than alpha. Preferably, barrier 94 will be adapted to provide a narrow channel or chamber 96 to increase the effectiveness of the wash fluid as the microparticles find their way under centrifugal force to read chamber 95. In the embodiment shown, the wash fluid serves also as the substrate solution. The sample mixture may be applied to the sample reservoir chamber 98 through orifice 91 and similarly wash fluid added to reservoir 97 through orifice 92. The volumes of the chamber and wash fluid are appropriately chosen in accordance with the physical dimensions of carousel 100 and as economics dictate. For instance, the sample may be adjusted to 0.25 ml, the wash fluid to 1.2 ml and the chambers 96 and 95 designed to hold 0.3 and 0.9 ml respectively.

In operation, the first step would be to rotate carousel 100 appropriately to force the wash fluid over the top of barrier 94 to fill chambers 95 and 96. This intermediate speed is preferably chosen to allow the wash fluid to escape up the ramp of barrier 94 but not allow the sample to escape over the top of barrier 103 because of the steeper angle alpha. Figure 10 then illustrates the initial position of the fluids under an increased rotation sufficient to permit the sample to overflow barrier 103. As illustrated, the wash/reaction fluid fills chambers 95 and 96 and preferably a small area 105 in order to further limit physical mixture of sample 98 with the wash solution in chamber 96. As may be readily appreciated, small channels 106 effectively reduce turbulence and aid in limiting mixing of various fluids during the stages of operation. Under continuous centrifugal force, microparticles found within sample 98 are forced past small channels 106 through wash chamber 96 and eventually into substrate chamber 95. As previously described, the presence of the microparticles may be detected optically through windows 93. It is equally contemplated that other forms of labels and detection not optically restricted may be employed to detect the presence of labeled microparticles.

It will be apparent to the skilled worker in this field that various modifications and embodiments of this invention can be made without departing from the spirit and the scope thereof.

CLAIMS:

1. A microparticle separating, washing and reading device wherein microparticles are separated from a sample-immunoassay mixture, washed by one solution and then passed into a reading area wherein the microparticles may be reacted with a substrate solution, said device comprising:

a) a substantially circular carousel having a center and a plurality of radially dispersed chambers, said chambers having a closed end distal to the center and an open end proximal to the center for communicating with a feed area disposed substantially at the center, said carousel adapted for rotation about its center by a centrifuge;

b) barrier means, disposed substantially perpendicular to the radius of the carousel within said chambers and communicating with the walls of the chamber, for stabilizing the solutions; and

c) sample holder means, adapted for placement substantially at the center, for containing the microsphere sample-immunoassay mixture and dispensing the mixture to the chambers when sufficient centrifugal force is applied.

2. The device as described in Claim 1 wherein said wash solution has at least one component for adjusting the density of said wash solution selected from the group consisting of sodium chloride, glycine, sugars, serum albumin, natural polymers, natural copolymers, synthetic polymers, synthetic copolymers, dextran, and Ficoll".

3.  The system as described in Claim 1 wherein said wash solution is serum albumin selected from the group consisting of animal serum albumin and human serum albumin.

4.  A method for separating, washing and detecting microparticles employed in an immunoassay for determining the presence of an immunological determinant in a sample comprising the steps of:

providing a substantially circular carousel having a center and a plurality of radially dispersed chambers with closed ends distal to the center of the carousel and open ends proximal to the center of the carousel for communicating with a feed area disposed substantially at the center, said carousel adapted for rotation about its center by a centrifuge;

rotating the carousel;

adding to said feed area a substrate solution whereby the substrate solution is substantially evenly dispersed among the chambers and flows to the distal ends;

further adding to said feed area a wash solution having a density at the maximum equal to that of the substrate whereby a wash layer is formed in the chambers intermediate the substrate and the proximal end;

reacting the sample with microparticles coated with an immunological counterpart to the determinant to be assayed under conditions appropriate for an immunological reaction to form activated microparticles, said sample without microparticles having a density not more than that of the wash solution and said microparticles having a density at least equal to the substrate solution;

introducing the reacted sample and microparticles to the feed area;

subjecting the microparticles in the sample to appropriate centrifugal force whereby a third layer is formed in the chambers intermediate the wash solution and the proximal end and the microparticles are caused to move through the wash solution and into the substrate solution; and

measuring the presence of activated microparticles for quantitating the presence of the immunological determinant in the sample.

5. The method as provided in Claim 4 wherein the measuring step further comprises the steps of:

providing a substrate in the substrate solution which, when reacted with activated microparticles, forms detectable product;

allowing the activated microparticles to react with the substrate under appropriate conditions to form detectable product; and

measuring the presence of detectable product for quantifying the presence of immunological determinant in the sample.

6. The method as provided in Claim 4 or 5 wherein the step of reacting the sample with coated microparticles takes place in sample holder means adapted for placement on the carousel substantially within the feed area; and the step of introducing the reacted sample and microparticles further comprises placing the sample holder in the feed area and increasing centrifugal force whereby the sample and microparticles are added to the chamber.

7. The method as provided in Claim 4, 5 or 6 wherein said wash solution has at least one component for adjusting the density of said wash solution selected from the group consisting of sodium chloride, glycine, sugars, serum albumin, natural polymers, natural copolymers, synthetic polymers, synthetic copolymers, dextran, and Ficoll™.

8. The method as provided in Claim 4, 5 or 6 wherein said wash solution is serum albumin selected from the group consisting of animal serum albumin and human serum albumin.

9. A method for separating, washing and detecting labeled particles from a determinant containing sample-reagent immunoassay mixture comprising the steps of:

a) providing a carousel comprising a plurality of radially dispersed chambers having at the ends distal to the center at least one optical window, and at least one port in said chamber distal to the optical window for adding fluids to said chamber, said chamber further adapted to promote layering of said fluids when rotated;

b) adding to said carousel a wash solution having a density at least equal to that of the sample reagent mixture but less than that of the particles to be separated;

ORD-39

c) rotating said carousel to force the wash solution, by centrifugal force, to the end of the chamber distal from the center of the carousel;

d) adding to said chamber a sample-reagent mixture containing particles to be detected so that while the carousel is being rotated sample reagent and wash layers are formed;

e) further rotating said carousel about its center under conditions sufficient to move the particles to be detected through the wash solution to the optical window area; and

f) detecting labeled particles whereby the presence or absence of a determinant within the sample may be correlated.

10. The method as provided in Claim 9 wherein said wash solution has at least one component for adjusting the density of said wash solution selected from the group consisting of sodium chloride, glycine, sugars, serum albumin, natural polymers, natural copolymers, synthetic polymers, synthetic copolymers, dextran, and Ficoll".

11. The method as provided in Claim 9 wherein said wash solution is serum albumin selected from the group consisting of animal serum albumin and human serum albumin.

12. The method as provided in Claim 9, wherein said detecting labeled particles step comprises illuminating said labeled particles and detecting resultant effects selected from the group consisting of fluorescence, light scatter reflectance, and light absorbance.

13. The method as provided in/Claims 9/wherein the wash
solution further comprises a substrate and labeled
particles are detected by the conversion of substrate to a
detectable product.

any one of    to 12

14. The method as provided in Claim 4 wherein said
measuring the presence of activated microparticles step
comprises illuminating said microparticles and detecting
resultant effects selected from the group consisting of
fluorescence, light scatter reflectance, and absorbance.

ORD-39

FIG-1

0106536

1/3

FIG-2

FIG-4

FIG-3

# FIG-6

# FIG-7

# FIG-5a

SAMPLE, MICROSPHERES, LABEL

# FIG-5b

Substrate Solution Addition

# FIG-5c

Wash Solution Addition

# FIG-5d

Sample Mixture Addition

# FIG-5e

Separating, Washing And Reading

LIGHT SOURCE

FIG-8a

3/3

FIG-8b

CENTRIFUGAL FORCE

FIG-8c

FIG-9

FIG-9a

FIG-10